# EUROPEAN PATENT SPECIFICATION

(11) **EP 1 461 093 B1**
(45) Date of publication and mention of the grant of the patent: **03.10.2007**
(21) Application number: 02781029.0
(22) Date of filing: 02.12.2002
(51) Int. Cl.: A61L 27/38, A61L 27/22, A61L 27/50, A61L 27/60

(54) **MILK PROTEIN BIOFILM AND USES THEREOF**
MILCHPROTEINBIOFILM UND VERWENDUNGEN DAVON
FILM BIOLOGIQUE EN PROTEINES DU LAIT ET SES UTILISATIONS

(30) Priority: 30.11.2001 US 334068 P
(43) Date of publication of application: 29.09.2004
(73) Proprietor: UNIVERSITE LAVAL, Québec, Québec G1K 7P4 (CA)
(72) Inventor: SUBIRADE, Muriel, Sillery, Québec G1S 3J5 (CA); ROUABHIA, Mahmoud, Cap-Rouge, Québec G1Y 2C6 (CA)
(74) Representative: Guerre, Dominique
(86) International application number: PCT/CA2002/001857
(87) International publication number: WO 2003/045459

(56) References cited:
- WO-A-01/74928
- WO-A-95/15352
- DE-A- 2 422 308
- US-A- 5 514 379
- AUGUSTIN C ET AL: "A SKIN EQUIVALENT MODEL FOR COSMETOLOGICAL TRIALS: AN IN VITRO EFFICACY STUDY OF A NEW BIOPEPTIDE" SKIN PHARMACOLOGY, S. KARGER, BASEL, CH, vol. 10, no. 2, 1997, pages 63-70, XP000889816 ISSN: 1011-0283
- LEFEVRE T ET AL: "Conformational rearrangement of beta-lactoglobulin upon interaction with an anionic membrane" BIOCHIMICA ET BIOPHYSICA ACTA. PROTEIN STRUCTURE AND MOLECULAR ENZYMOLOGY, ELSEVIER, AMSTERDAM,, NL, vol. 1549, no. 1, 10 September 2001 (2001-09-10), pages 37-50, XP004305695 ISSN: 0167-4838

## Description

### TECHNICAL FIELD

The present invention relates to membrane or biofilm composition and method of making and using the same. The biofilm composition is preferably a membrane or lamina. The invention also relates to the culturing of mammalian support dependent cells onto a substrate, a cell support lamina. More particularly, relates to the formation of lamina formed with biocompatible non-immunogenic graft membranes.

### BACKGROUND OF THE INVENTION

Different cells, such as epithelial cells or keratinocytes, are support dependent. Such cells cultured in the presence of a substrate which is non-inhibitory and non-cytotoxic will multiply in stratified colonies and eventually produce a confluent layer. Cell cultures of this type are used to investigate skin growth and have been used as skin grafts. Various technical papers have been published which describe *in vitro* techniques for growing skin cells and their subsequent use in the treatment of full-thickness wounds (E. Bell et al, J Invest Derm 81; 2s-10s 1983; E. Bell et al, Science 211; 1052-1054 1981; and J. F. Burke et al, Ann Sura 94; 413-428 1981).

The ability of cells to adher to a particular substrate is dependent on the properties of the substrate itself as well as the culturing conditions and the components of the culture medium. Culturing is usually carried out in hard plastic flasks made from a material which is substantially inert to the growth media and is non-cytotoxic to the cells. Polystyrene is a commonly used material for culture flasks.

One of the problems in using hard plastic flasks for the culture of epithelial cells for use as skin grafts is that the sheet of cells normally has to reach confluence before they can be harvested. The time taken to reach confluence may be long. Furthermore the layer of cells is not very mechanically strong and can be easily damaged when the unsupported cells are dislodged and handled unsupported.

It is recognized in the art that a sheet of cells can be supported after it has been dislodged from the surface on which it was produced in order to facilitate the handling and transfer to a body surface. However this technique does not overcome the problems associated with hard surface cultures or the risks associated with dislodgment of the cells.

Several types of cell have also been grown on natural materials such as collagen which can then be used directly as, a skin replacement. Whilst these cells can grow on collagen, there are several disadvantages in using such a material. Since collagen is a natural substance, it is not completely defined and can vary substantially from one batch to another, a fact that is clinically undesirable if it is for use as a skin graft. Collagen is also a material difficult to work with in a laboratory and it is a complex and time consuming process to isolate it, making it expensive to produce. A further disadvantage is that because collagen is a protein it cannot be easily sterilized by methods such as steam penetration as it will be denatured. Accordingly it is difficult to store and to keep sterile.

PCT application published at No. W088/08448. describes the use of collagen and hard plastic surfaces as cell culture substrates it has been proposed to grow cells on the surface of water-swellable hydrophilic synthetic polymers. The cells are not immersed in the aqueous medium but are grown at the substrate-gaseous interface. The resultant sheet is relatively bulky making it poorly conformable and the cell layer has to be fully confluent before it can be transferred to the wound site which can take a considerable time, as for example between 14-21 days.

Also, while there exist biocompatible macromolecular skin graft, or tissue substitute compositions described in the literature, an impediment for *in vivo* implantation (both cutaneously and in any degree more internal than cutaneous, e.g., subcutaneous or intraperitoneal application) of biocompatible implants suffers of lack of reliability. Another deficiency of these implants is that they are often not retrievable after implantation. This deficiency is significant, particularly when combined with yet a further deficiency of these implants, namely that they can lose there physical properties. For instance, implants made of biodegradable materials degrade over time and thereby induce a local or a systemic immune response in a host system. Further, another deficiency of such implants is the difficulty to monitor and inspect such implants.

Different approaches have been taken to improve the biocompatibility of implantable items. One approach was to prevent undesirable protein adhesion by providing a biomaterial with a low polarity surface, a negatively charged surface, or a surface coated with biological materials such as enzymes, endothelial cells, or proteins. Another approach was to coat solid surfaces with heparin, albumin, or streptokinase to enhance the thromboresistance. However, these approaches have failed to show a covalent binding.

Considering the state of the art, it would be highly desirable to be provided with a non-immunogenic, biocompatible and biodegradable biofilm for the preparation of tissue constructs, tissue grafts, or replacement cell's sheet.

### SUMMARY OF THE INVENTION

One object of the present invention is to provide a method for the *in vitro* culture of support dependent mammalian cells to produce tissue construct, said method comprising the steps of:
contacting an aqueous culture medium containing an aqueous culture medium containing support dependent mammalian cells with a biofihn comprising milk proteins and a plasticising quantity of at least one plasticizer; and
culturing the cells on the biofilm to form a cell layer adhered to the biofilm to form the tissue graft.

In accordance with the present invention there is provided a method wherein the biofilm comprises between 1 to 80% of milk protein and between 1 to 50% of plasticizer, and wherein the milk proteins may be at least one of a whey protein, a casein, a globulin, or a lactoglobulin.

Also the cells may be selected from the group of fibroblasts, keratinocytes, muscular cells, skin cells, epithelial cells, mesenchymal cells, mesodermal cells, and adult stem cells.

The plasticizer may selected from the group consisting of glycerol, diethyleneglycol, polyethylen glycol, polybasic acid, phosphoric acid, fatty acid or substitute thereof, palmitate, stearate, oleate, ricinoleate, laurate, sorbitan monolaurate, sorbitan monooleate, poly(oxyethylene)-(20)sorbitan monolaurate, butyl acetyl ricinoleate, glycerol tricaprolate, glycerol tributyrate, triethylene glucol caprylate, polyglycol, polyoxyalkylene, pentaerythritols, sulphonic acid derivative, epoxy derivative, chlorinated paraffin, polymeric ester, glycerol triacetate, and glycerol diacetate, or a derivative or a mixture thereof.

In accordance with the present invention there is provided a laminated tissue construct comprising at least one layer of biofilm comprising milk proteins and a plasticising quantity of at least one plasticizer, and at least one layer of cultured mammalian cells adhered on at least one side to said biofilm.

It is an object of the invention to provide a non-immunogenic, biocompatible biofilm and methods of making and using such.

Another object of the invention to provide such a laminated tissue construct having a biofilm as the membrane and immunogenic, biocompatible properties by virtue of milk proteins covalently bound at or to the surface of the membrane via at least one plasticizer.

It is yet an object of the invention to provide such a composition in the form of a sheet.

It is additionally an object of the invention to provide a surface or a space, such as on the exterior or within a composition either in sheet form for culturing cells, harvesting product therefrom or otherwise delivering, administering or secreting a substance *in vitro* or *in vivo* as well as to provide methods employing such.

It is still another object of the invention to provide an implant or transplant system which exhibits a suitable level of biophysical stability or biocompatible devices after implantation or transplant.

It is yet another object of the present invention to provide a composition exhibiting suitable longevity and/or flexibility for instance, for use in transplant, implant, biocompatible device, or culturing or harvesting.

It is yet a further object of the present invention to substantially avoid an elicited immune response by an antigen or immunogen by supporting either cells or product therefrom in a biocompatible biofilm.

It is also an object of the present invention to provide a biocompatible laminated tissue construct, such as a biocompatible replacement living tissue composition in sheet form, that can adher itself or be adhered in a host organism or, to provide a adher means for supporting implanted cells, for adhering them within an laminated tissue construct or within a host.

It is additionally an object of the invention to provide an *in vivo* system capable of controlling or treating or preventing a medical condition in an individual in need thereof by secretion or administration by osmotic or other relative concentration driven mechanism of a substance for such control or treatment or prevention from secretion by implanted cells expressing the substance or from the substance encapsulated in a permeable, enclosed, membrane system that is preferably non-immunogenic and/or biocompatible.

### BRIEF DESCRIPTION OF THE DRAWINGS

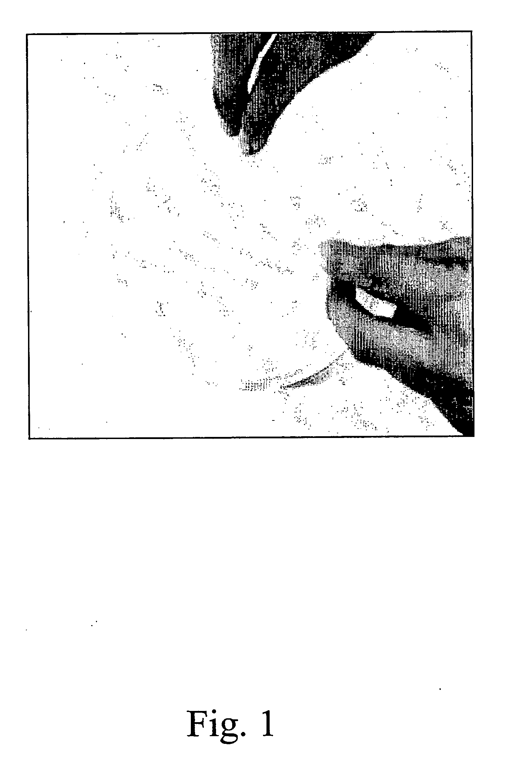

Fig. 1 illustrates a biofilm according to one embodiment of the invention;

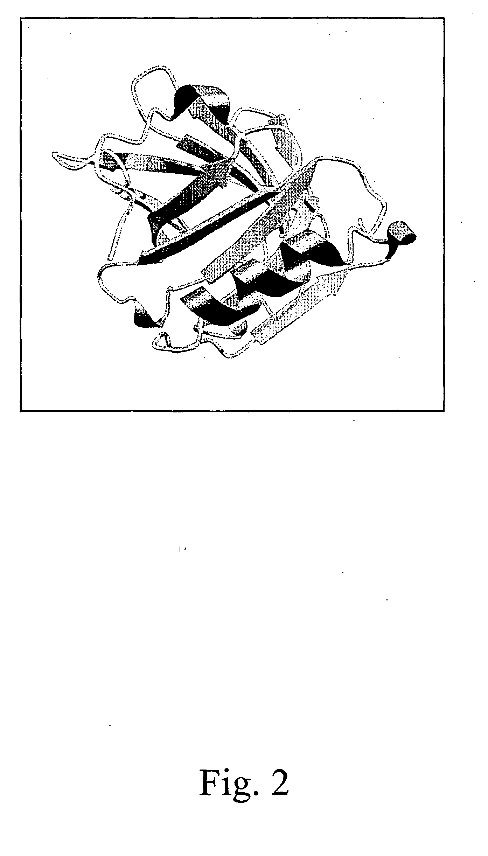

Fig. 2 illustrates the tridimentional molecular aspect of the native β-lactoglobulin ;
Fig. 3 illustrates an infra-red spectrum of the a biofihn according to the invention;
Fig. 4 illustrates the effect of the type and concentration of the plasticizer in the (left scale = MPa, and right scale is σR (MPa) ;
Fig. 5 illustrates the effect of the concentration of the plasticizer (ethylen glycol) in the (left scale = MPa, and right scale is σR (MPa);
Fig. 6 illustrates the proliferation level of keratinocytes grown on a biofilm according to one embodiment of the invention;

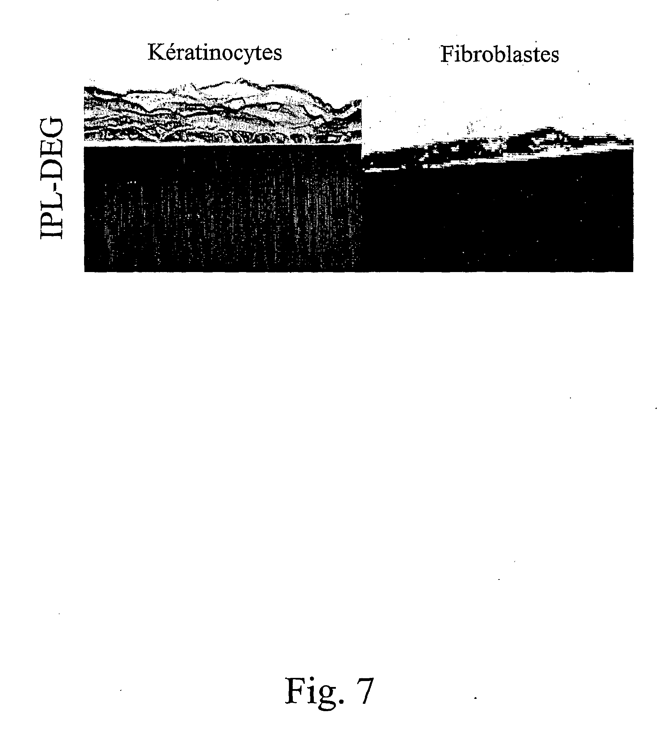

Fig. 7 illustrates the structural organization of skin cells;

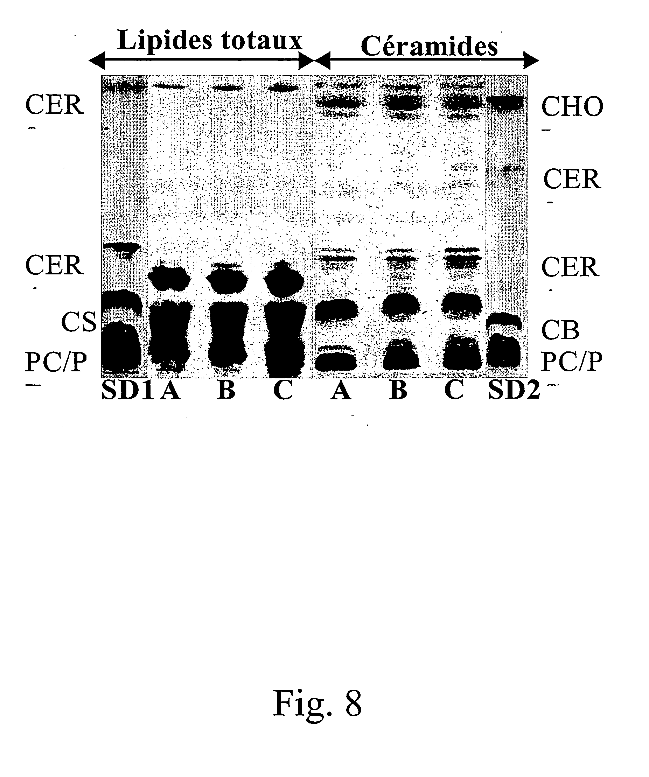

Fig. 8 illustrates the influence of a biofilm of the invention on the lipid composition of cutaneous tissues; and

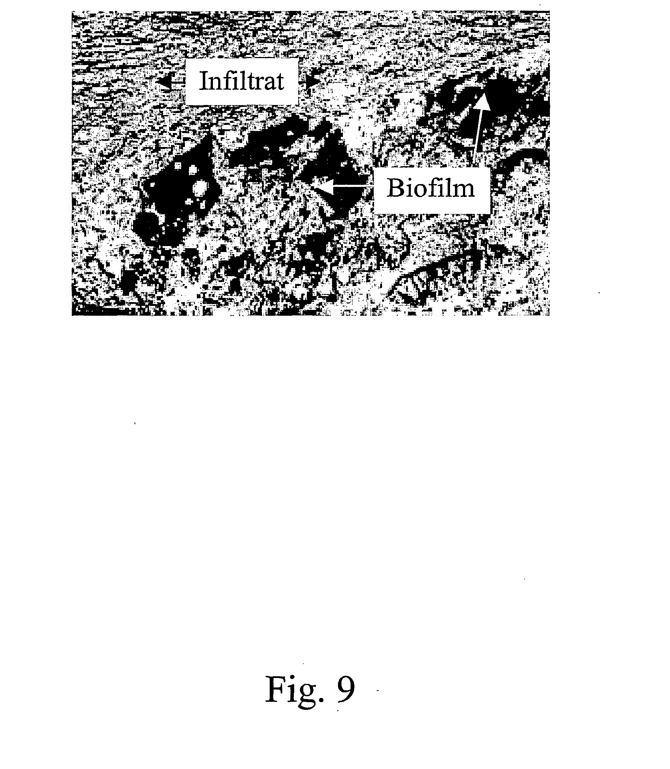

Fig. 9 illustrates the *in vivo* biodegradability of the biofilm of the invention.

### DETAILED DESCRIPTION OF THE PREFERRED EMBODIMENT

The present invention relates to a non-immunogenic and biocompatible biofilm and its preparation and use. The biofilm of the invention is characterized as being "biocompatible" if is capable of functioning and/or existing in contact with biological fluid and/or tissue in a living organism with a net beneficial effect without eliciting an immune response in the living organism. Long term biocompatibility is desired for the purpose of reducing disturbance to the host organism if used *in vivo,* as well as to provide products over a period of time when used *In vitro*. In the present invention, at least one milk protein, and most preferably β-lactoglobulin, is used to make the biofilm biocompatible and biodegradable.

The biofilm of the present invention is characterized by a composition avoiding to induce undesirable reactions in the body (such as blood clotting, tissue death, tumor formation, allergy reaction, immune response or an inflammatory reaction), has desirable physical properties (such as strength, elasticity, permeability, and necessary flexibility, also be purified, fabricated, and sterilized easily) and is able to maintain its physical properties and functions during the time the membrane composition remains implanted in or in contact with the host body.

According to another embodiment of the present invention, there is provided a method for producing a laminated tissue construct by culturing support dependent mammalian cells adhered to a biofilm comprising milk proteins and at least one plasticizer, which is non-inhibitory to cell growth, non-cytotoxic, together with means for maintaining an aqueous culture mechanism containing the cells in contact with one surface of the biofilm.

The biofilm acting as substrate in a laminated tissue construct is generally not be inhibitory to cell growth. A measure of such inhibition can be expressed as a percentage cell growth reduction as measured against cells allowed to grow in the absence of a test substrate as hereinafter described. The substrate biofilm does result in no more than 50% reduction in cell growth. More aptly it should not result in more than a 40% reduction in cell growth. It preferably not results in more than a 20% reduction in cell growth relatively to cells grown in the same conditions without the substrate biofilm.

The biofilm has low cytotoxicity, and normally no toxicity of negative effects relating to cell growth. The cytotoxicity can be measured against a non-cytotoxic control material by a method as hereinafter described.

The biofilm may be in the form of a continuous film or an apertured film, for example, formed into a net. The substrate biofilm can be a continuous film which is adapted to be perforated after the cell layer has formed a sheet, at least partially, on the substrate biofilm.

Preferably the biofilm is transparent enough to allow the visualization of the body site against which it is applied.

It is preferred to use autologous cultivated cells in the preparation of a laminated tissue construct, such as, without limiting it to, epithelial cells or keratinocytes, for example, since these have little or no immunological rejection problems when applied to the host.

The cells can have reached different levels of confluence before transfer. It is possible, when needed, that a suitable laminated tissue construct be produced within a few hours making the tissue construct particularly suitable for use as an emergency field dressing when rapid treatment is required.

Depending of the needs, the cellular sheet comprises a cell layer which is not more than 2 cells thick, more preferably the cell layer is a monolayer.

The biofilm can be sterilized by either ethylene oxide (allowing the required time for de-gassing), alcohol or by gamma-irradiation. It is important that the biofilms are washed to remove any low molecular weight contaminants, for example milk contaminants. Such contaminants can be cytotoxic and for the reasons given above the biofilm should be substantially non-cytotoxic. The washing process may comprise several sequential washes using sterile de-ionized water in sequential steps.

In the laminated tissue construct of the present invention, the biofilm on which the cells are grown can be generally easily removable from the other parts of the system, namely the means for maintaining the aqueous culture medium containing said cells in contact with one surface of the film. For example, where the biofilm forms one of the walls of the vessel containing the culture medium, it should be readily removable from the other parts of the vessel. The biofilm may form a part or all of the container in which the cell culture is grown. The other parts of the container or culture vessel may be formed from suitable materials conventionally used for the manufacture of tissue culture vessels.

In an alternative embodiment the biofilm may be laid down within a flask of an appropriate design adapted to allow the removal of the substrate biofilm. Where the substrate is an integral part of the culture flask it may be removably sealed to the other parts of the flask for example by heat sealing or by means of an adhesive. It will be recognized by someone skilled in the art that the biofilm can form a flat surface and can also form the wall of the flask.

Where the biofilm is to be contained within a flask, the flask can be provided with a collapsible opening having dimensions sufficient to enable the biofilm to be readily removed without disruption of the cells adhered thereto.

A biofilm of the present invention when used as a cell-supporting membrane can allow to obviate the problems associated with the use of cultured multilayer epithelial cell sheets. For example, culturing cells on the biofilm provides a support for the cells such that the handling of the material to be grafted, for example, is facilitated. In addition, the use of such a support allows the graft to be transferred to the body site a short time after the membrane is seeded with the cells.

Specifically, such grafts are suitable for application to a wound about one day to about seven days after seeding, at a time when the cells are not yet confluent. Since the cells are subconfluent when the graft is applied to the body site, the cells, such as keratinocytes, primarily may be in the proliferative stage of growth and, therefore, express the appropriate cell surface adhesion molecules.

It will be recognized that the invention is particularly useful when a biofilm contains a subconfluent monolayer of keratinocytes because the keratinocyte-supporting membrane can be applied to the body site, as for example, but not limited to, a wound, a short time after the wound is incurred. However, where time allows, the keratinocytes can be cultured to confluence or, if desired, can be grown to form a multilayered epithelial cell sheet of about two to four layers on the biofilm, which provides a support for the cells and allows their transfer to the wound without the need for enzymatic or other treatment of the keratinocytes.

The present invention also provides cells that are cultured on the surface of a biofilm. This can form a laminated tissue construct, comprising at least one layer of cells and at least one layer of biofilm. The support membrane then is inverted over the body site such that the surface of the cells that initially was exposed to the medium, i.e., not attached to the biofilm, ultimately becomes apposed to the body site surface. Thus, the polarity of the cells must reverse upon their transfer from the culture medium to a wound.

In one embodiment of the present invention, the laminated tissue construct may be used as replacement living tissue, as for wound dressing for example, or for skin layer replacement.

### EXAMPLE I

### Preparation and characterization of a biofilm

### Materials and methods

Whey protein isolate (WPI) and B-lactoglobulin (BLG) were obtained from Davisco (Food International, Inc, Le Sueur, Minnesota). The WPI contains 92.5% (dry matter basis) proteins with 78% of BLG, 16.6% of α-lactalbumin (ALA) and 5.4% of Bovine serum albumin (BSA). BLG is a purified fraction of WPI (>92%) obtained from fresh, sweet dairy whey, using a unique ion-exchange technology. The enzymes used in the study were pepsin 1:60 000 from porcine stomach mucosa, crystallised and lyophilised, (Sigma Chemical Company St-Louis, MO, USA) and pancreatin 5X from hog pancreas (ICN Nutritional Biochemicals Cleveland, OH, USA). Thimerosal (J. T. Baker, Phillisburg, NJ, USA) was used to prevent bacterial growth and taurocholic acid, sodium salt form, (Sigma Chemical Company St-Louis, MO, USA) is an emulsifying agent. Plasticisers agents -Glycerol (GLY), ethylene glycol (EG) and diethylene glycol (DEG)- were of analytical grade.

### Whey proteins biofilms preparation

Film-forming solutions were prepared by dissolving whey protein isolate (WPI)/ or B-lactoglobulin (BLG) in distilled water (10 %w/v) according to the following method. After 1h30 of mixing with a magnetic stirrer, the pH value was adjusted to 7. Subsequently film-forming solutions were conditioned in a water bath at 80° C for 30 min to denature the protein and cooled down to 23° C. After 1h of mixing, the plasticizer was added with a protein/plasticizer ratio equal to 1. The stirring was continued for 30 min to form an uniform solution. The solution was then centrifuged to eliminate the air bubbles (2300 rpm, 10 min). The solution was cast into Petri dish of 140 mm in diameter and the solvent evaporated in an oven for 20h at 23°C and 43% RH produced by a saturate solution of K₂CO₃. After drying, an uniform film was obtained. The film was peeled from the Petri dish using a spatule and stored (T:23°C and RH:43%) to await further use. Using this procedure, different biofilms were obtained: WPI biofilms in the presence of EG (called in the follow WPI/EG), in the presence of DEG (called WPI/DEG) and in the presence of GLY (called WPI/GLY) and BLG biofilm in the presence of DEG (called BLG/DEG).

### Mechanical properties

An Instron universal testing instrument Model 5565 equipped with a 50 N strength captor was used to measure the tensile properties of films according to ASTM standard method D882-91. Initial grip separation and crosshead speed were set at 50mm and 24 mm/min, respectively. The tested film strips were 80 mm long and 6.35 mm wide. Stress elongation curves were recorded during extension and stress at break (σ_{b}), strain at break (ε_{b}) and Young's modulus (E) were calculated. Tensile values were reported as averages of 7 replicates for each film type. Before testing, the thickness of the films was measured at ±0.001 mm by a digital micrometer at five random positions along the rectangular strips. The mechanical properties were determined using the average thickness for each film replicate.

### In vitro enzymatic degradation studies

The susceptibility of biofilms to proteolytic enzymes was determined using two proteolytic enzymes (pepsin and pancreatin) as controls because of their presence in organism.

Peptic degradation: each sample of 260mg of film powder was suspended in 20 ml of 0.1N HCL which contained 50ppm of thimerosal to the micro-organisms growth. After 10 min magnetic stirring at 37C, the pH was ajusted at 1.9 with HCl and a pepsine solution (1mg/ml prepared in 0.1NHCL) was added at an enzyme: substrate mass ratio of 1:250. At different time intervals, 2ml samples were collected, mixed with an equal volume of 20% trichloracetic acid to precipitate protein fractions above 10 000 Da and centrifugated for 15 min at 15000g. The supernatant (TCA soluble material) was sampled and the nitrogen contents of TCA soluble materials were measured using autoanalyzer (ANTEK 7000 nitrogen/sulfur analyzer, ANTEK instruments, Inc., Houston, USA). Control samples were incubated under similar conditions but without enzyme. Each experiment was performed in triplicata.

### Pancreatic degradation

For the determination of pancreatin sensitivity, the same procedure was used but the pH of the reaction was 7.5 and a pancreatin solution (10mg/ml prepared in a phosphate buffer 0.01M) was added at an enzyme: substrate ratio of 1:25.

### Toxicity experiments

The toxicity of different biofilms (EG/WPI, DEG/WPI, GLY/WPI, DEG/BLG) were assessed using normal human keratinocytes and fibroblasts. Skin donors were healthy women, 15 to 20 years of age. Cells were isolated from skin biopsies following breast reductive surgeries as previously described. Following their isolation, both cell types were seeded into 75 cm² culture flasks (Falcon, Becton Dickinson, Lincoln Park, NJ, USA). Keratinocytes (9 x 10³cells/cm²) were cultured with 1.3 x 10⁴/cm² irradiated mouse 3T3 fibroblasts in Dulbecco's modified Eagle (DME) with Ham's F12 (DMEH) (Flow Laboratories, Mississauga, ONT ) supplemented medium: 5 µg/ml insulin, 5 µg/ml human transferrin, 2 x 10⁻⁹ M 3,3',5' triiodo-L-thyronine (Sigma), 0.4 µg/ml hydrocortisone (Calbiochem, La Jolla, CA), 10⁻¹⁰ M cholera toxin (Schwarz/Mann, Cleveland, OH), 10 ng/ml EGF (Chiron Corp. Emeryville, CA), 100 UI/ml penicillin G (Sigma), 25 µg/ml gentamicin (Scheering Inc., Pointe-Claire, CAN) and 10% newborn calf serum (Hyclone, Flow Laboratories). Fibroblasts (1 x 10⁴ cells/cm²) were grown in Dulbecco's modified Eagle (DME) supplemented with 100 UI/ml penicillin G (Sigma), 25 µg/ml gentamicin (Scheering Inc.) and 10% fetal calf serum (Gibco, Burlington, CAN). The media of both cell types were changed three times a week. When the cultures reached 70-80% confluence for keratinocytes and 100% for fibroblasts, the cells were detached from the flasks with 0.05% trypsin-0.1% EDTA solution, washed twice and then resuspended in DMEH or DME-supplemented media at a final concentration of 10⁶cells/ml. These cells were then used to test the toxicity of each biofilm. For this purpose, disks (9 cm²) were prepared from each biofilm then sterilized by 3 x 5 min washes with an alcohol solution (90 % ethanol-10 % distiled water), followed by 3 x 2 min washes in sterile culture medium. These sterilized disks were introduced into 6 well plate then seeded with previously isolated fibroblasts (50 00 cells) or keratinocytes (100 00 cells). As controls, keratinocytes and fibroblasts were also cultured separately using 6 well plates (Falcon). Cells were grown under submerged conditions at 37°C in a humidified atmosphere containing 8 % CO₂. After being cultured for 1, 2, 3 4 or 5 days, the cells were trypsinized, resuspended in 1 ml of cultured medium, and then 1 ml trypan bleu was added to each cell suspension. Viable (trypan blue exclusion) keratinocytes and fibroblasts in each sample were counted using hemacytometer under an optical microscope. Experiments were repeated three times, then viable cells were plotted as means ± SD. Comparisons of .cell number between control and biofilms were made by using the Student's *t* test. Results were considered significant if p < 0.05.

### Results

### Mechanical properties of biofilms: effect of plasticizer and film-forming materials.

The formulation of protein-based films usually requires the addition of a plasticizer to overcome brittleness and rigidity and to induce sufficient flexibilty and extensibility. As demonstrated herein, the plasticizers enhance the mechanical properties of biofilms by reducing the too extensive intermolecular interactions within plopeptides chains responsible for cracking during the drying process, thereby increasing the mobility of protein chains.

In this work, different plasticizers have been used in order to obtain a large range of materials. In the first time, the effect of ethylene glycol (EG), diethylene glycol (DEG) and glycerol (GLY) on the mechanical properties of the films were compared using Whey protein isolate (WPI) as film-forming materials. In the second time, mechanical properties of β-lactoglobulin (BLG) and whey protein isolate (WPI) films were compared using DEG as plasticizer.

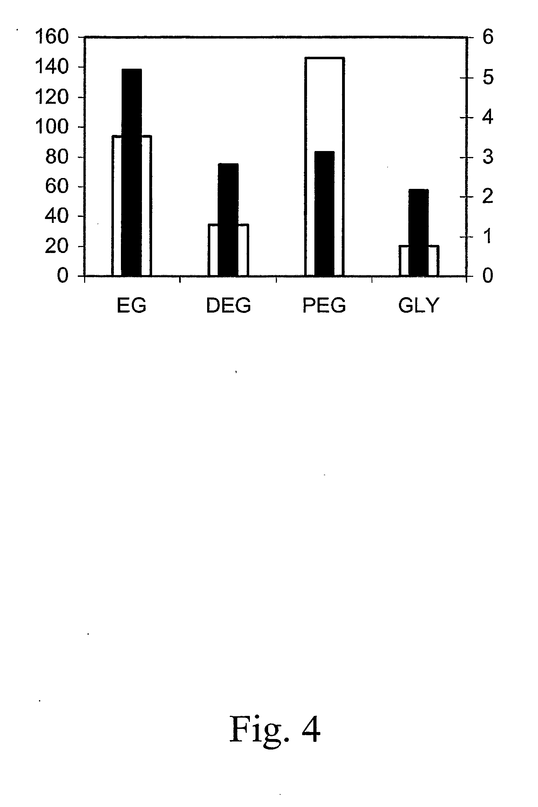

Fig. 4 shows typical static stress-strain curves. The values of Young's modulus E, the stress at break (σ_{b}), and the strain at break (ε_{b}) as a function of plasticizer and film-forming materials are summarized in Table 1.

Considering WPI biofilms, one can see that the E values, (σ_{b}) and (ε_{b}) decrease following the order EG> DEG> GLY, suggesting a plasticizer dependence of mechanical properties. This effect is well known. By their insertion into the protein chains, plasticizers reduce the intermolecular protein-protein interaction and increase intermocular spacing between chain, favoring the plasticizer-protein interaction and thus modify the mechanical properties of biofilm. This effect is modulated by the plasticizer structure, i.e. alkyl chain length, alkyl chain branching, separation of ester groups and additional polar groups of the plasticizer structure. Considering the chemical structure of plasticizers used, EG is a small hydrophilic diol which can be easily inserted between protein chains allowing to their hydroxyl groups establish hydrogen bonds with reactive groups of unfolding pre-heated protein molecules. This has as a consequence to lead to the formation of closer alignment of polypeptide chains with the formation of much stronger hydrogen bonds compared to DEG which is also a diol but with an higher alkyl chain length. Moreover, on the glycinin-biofilms, it is observed that EG can promote the orientation of polypeptide chains leading to better mechanical properties compared to other plasticizers. In the case of GLY, it can be comprised that the presence of three hydroxyl groups able to interact with functional group of protein can lead to a less organized network and to poorer mechanical properties.

With regards to the influence of the whey protein purety (WPI versus BLG), it could have been expected that the BLG and WPI-based materials would show similar results because of the dominating protein β-lactoglobulin. Using the purified major protein instead of isolate apparently allows to obtain more resistant (higher σ_{b}) and more extensible films (higher ε_{b}). The formation of more resistant films may be due to changes in the type, density and reversibility of intermolecular interactions occurring in the whey protein network that forms the films.

The major forces usually involved in the whey protein network are van der Wals interactions, hydrogen bonds, electrostatic interactions, and S-S bonds. In the case of BLG alone, it can be recognized that intermolecular-disulphide bonds are formed and also sulphydryl/disulfide bond exchange reactions occur. In the latter reaction, the free, highly reactive -SH groups of cys 121 in each monomer has been shown to be involved in intramolecular and intermolecular disulfide interchange with other -SH group which may be responsible to the higher mechanical properties obtained.

In the case of WPI, the SH groups come mainly from BLG because WPI contains only little amount of BSA with a free thiol group Cys 34, and ALA does not contain any SH group. Eventhough it can be taken into account that when mixtures of BSA and BLG are heated at 80°C and examined at room temperature the gels from the mixture are stronger, recent results shows that this effect is dependent on the composition of the mixture and the presence of BSA in a BSA/BLG ratio less than one has as a consequence to stabilize the native structure of BLG.

From the present results, it can be seen that it is possible to modify considerably the mechanical properties of the films symply by changing the type of plasticizer and the protein fraction such as milk proteins. The ability to modulate the mechanical properties of films is of primary importance for the preparation of biomaterial which must fulfill specific mechanical requirements.

### Biological test

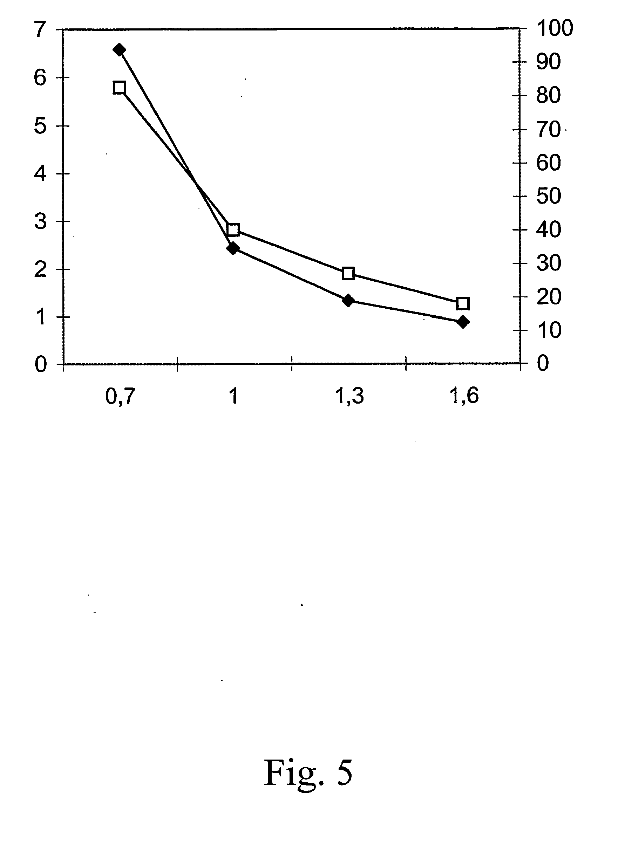

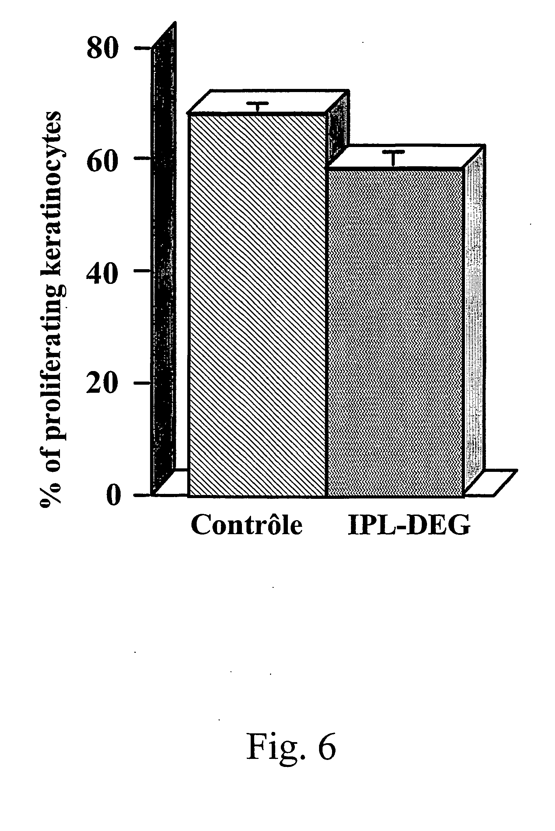

The main purpose of this study was to evaluate the compatibility of different biofilms with living mammalian cells. A cell growth and viability assay was performed on human keratinocytes and fibroblasts to investigate whether or not the whey protein-based biofilms were toxic. As shown in Figs. 5 and 6, the number of viable fibroblasts (Fig. 5) and keratinocytes (Fig. 6) following their culture onto biofilms was significantly (p < 0.01 ) less than the control, but still very interesting in term of viable number. Indeed, in fibroblast cultures, viable cells increased 45 to 80 folds when compared to the seeding concentration (from 5000 cells to 200-400 x 10³). The same observations were made for keratinocyte growth. If we compare the different biofilms, it is obvious to see that the keratinocyte growth was variable from one biofilm to another. At confluent time, keratinocyte numbers were classified as follow: DEG/WPI>EG/WPI>DEGBLG>GLY/WPI. Regarding the fibroblast growth, also, with the different tested biofilms, the cells had a growth dependency on the biofilm as follow: GLY/WPI>DEG/WPI>EG/WPI>DEGBLG. The results obtained with fibroblast growth were comparable to those obtained with keratinocytes suggesting that, DEG is compatible for both cell types with no toxic properties. Also EG and GLY biofilms were compatible for both cell types, but with less efficacy compared to DEG. However, in all cases, the cell growth onto biofilms was lower then cell growth onto specific culture dishes.

### Biodegradation

While considerable effort has been made to characterise whey-protein films, nothing has been shown before the present works particularly on their biological degradation such as enzyme-induced hydrolysis. The relative protease sensitivities of biofilm were evaluated using pepsin and pancreatin, two digestive enzymes. The WPI biofilm formed in the presence of DEG was taken as the model since it presents no toxic properties neither against fibroblasts nor for keratinocytes.

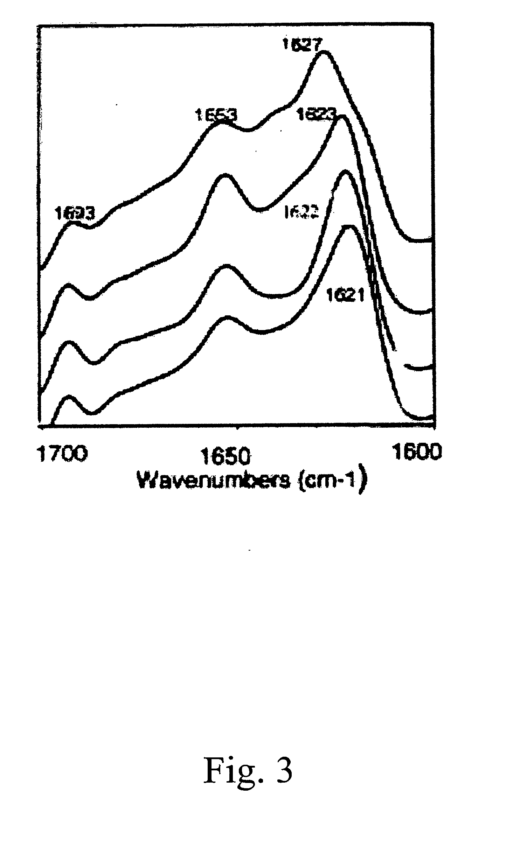

Fig.3 displays the biofilm degradation in the presence of pepsin (A) and pancreatin (B) and in the absence of enzyme. This figure illustrates the fact that whey protein films demonstrate significantly greater resistance to peptic degradation than to pancreatic degradation. The films are rapidly digested by pancreatin - about 100% after 4 hours-, whereas their degradation rate in pepsin is shown to be strongly reduced - about 20% after 4h and only 33% after 12h. We can be noted that without enzyme, there is not degradation of films suggesting that it is mainly governed by the enzyme protease. This implies that the residence time of the protein film might be controlled by changing the protein concentration of the film.

These results reveal that the biodegradation of whey protein films depend on the specificity of enzyme. Considering pepsin, it is known that this enzyme preferentially catalyses the hydrolysis of peptide bonds involving hydrophobic aromatic amino-acids. It can be considered that the rate of proteolysis of BLG is related to its conformation and that heat treatment of the protein above its denaturation temperature (above 80°C) rendered it accessible to pepsin. Since in the initial step of the formation of films, the protein molecules are heated above their thermal denaturation temperature leading to an exposure of their hydrophobic amino-acids, it was expected to a significant increase in the susceptibility of proteins to proteolysis degradation and specially to peptic digestion. However, enzyme-induced digestion is sensitive to changes that affect the conformation of substrates or the actives site of enzymes. Diffusion of the enzymes in the film network could be of importance, since it is the contact between the active site on the enzyme and the substrate that decides the degradation of the system. It can be supposed that the hydrophobic amino acids are trapped in the protein network and thus partially masked preventing the action of pepsin.

Concerning degradation by pancreatin, the films were completely destroyed within 4 hours. After this incubation time, nothing remain in the solution. This degradation to pancreatin would then be attributed to the combined effect of the proteases mainly trypsin, chymotrypsin and elastase which catalyse the hydrolysis of peptide (amine) bonds but with different specificities. It is known that the action of trypsin is restricted to peptide links involving the carboxylic groups of lysine and arginine; chymotrypsin is specific for a bulky hydrophobic residue preceding the scissile peptide bond, and elastase is specific for a small neutral residue. The combined effect of the proteolytic enzymes present in the pancreatin is more efficiency to degrade biofilm compared to pepsin alone.

It can therefore be concluded that the degradation of whey protein biofilms are enzyme-dependent. So, parameters such as the crosslinking density of network, the substrate conformation or protein concentration in the film could be modified to obtain the system with desirable degradation profiles making it more resistant to the enzymatic attack in an *in vitro* or *in vivo* context.

From these data, it is clear that whey proteins which are readily available, inexpensive, and from milk origin, appear to be a good substrate for biomedical applications. As a biomaterial, whey protein displays several new characteristics: it is a natural polymer which has very low or no antigenicity, it is completely resorbable *in vivo* and its physicochemical properties can be suitably modulated. The possibility to modulate these properties through variations in composition is of primary importance for the preparation of biomaterials which must fulfill specific mechanical requirements. Furthermore, due to the large number of functional side groups it contains, whey protein readily undergoes chemical cross-linking, which is very important for its possible use as a non-toxic biocompatible biomaterial.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features herein before set forth, and as follows in the scope of the appended claims.

## Claims

1. A method for the production of laminated tissue construct, comprising the steps of:
a) contacting an aqueous culture medium containing support dependent mammalian cells with a biofilm comprising at least one milk protein selected in the group consisting of a whey protein, a globulin, a lactoglobulin or fragments thereof and a plasticizing quantity of at least one plasticizer, and
b) culturing said cells on said biofilm in condition allowing the formation a cell layer adhered to said biofilm to form the laminated tissue construct.

2. the method of claim 1, wherein said biofilm comprises between 1 to 80% of milk protein and between 1 to 50% of plasticizer.

3. The method of claim 1, wherein said milk protein is a β-lactoglobulin.

4. The method of claim 1, wherein said cells arc selected from the group of fibroblasts, keratinocytes, muscular cells, skin cells, epithelial cells, mesenchymal cells, mesodermal cells, and adult stem cells.

5. The method of claim 1, wherein said plasticizer is selected from the group consisting of glycerol, diethyleneglycol, polyethylen glycol, polybasic acid, phosphoric acid, fatty acid or substitute thereof, palmitate, stearate, oleate, ricinoleate, laurate, sorbitan monolaurate, sorbitan monooleate, poly(oxyethylene)-(20) sorbitan monolaurate, butyl acetyl ricinoleate, glycerol tricaprolate, glycerol tributyrate, triethylene glucol caprylate, polyglycol, polyoxyalkylene, pentaerythritols, sulphonic acid derivative, epoxy derivative, chlorinated paraffin, polymeric ester, glycerol triacetate, and glycerol diacetatc, or a derivative or a mixture thereof

6. A laminated tissue construct comprising at least one layer of biofilm comprising at least one milk protein selected in the group consisting of a whey protein, a globulin, a lactoglobulin or fragments thereof and a plasticizing quantity of at least one plasticizer, and at least one layer of cultured mammalian cells adhered on at least one side to said biofilm.

7. The laminated tissue construct of claim 6, wherein said cultured mammalian cells form a sub-confluent cell layer or a confluent cell layer.

8. The laminated tissue construct of claim 6, wherein said biofilm comprises between 1 to 80% of milk protein and between 1 to 50% of plasticizer.

9. The laminated tissue construct of claim 6, wherein said milk protein is a β-lactoglobulin.

10. The laminated tissue construct of claim 6, wherein said cells are selected from the group of fibroblasts, keratinocytes, muscular cells, skin cells, epithelial cells, mesenchymal cells, mesodermal cells, and adult stern cells.

11. The laminated tissue construct of claim 6, wherein said plasticizer is selected from the group consisting of glycerol, diethyleneglycol, polyethylen glycol, polybasic acid, phosphoric acid, fatty acid or substitute thereof, palmitate, stearate, oleate, ricinoleate, laurate, sorbitan monolaurate, sorbitan monooleate, poly(oxyethylene)-(20) sorbitan monolaurate, butyl acetyl ricinoleate, glycerol tricaprolate. glycerol tributyrate, triethylene glucol caprylate, polyglycol, polyoxyalkylene, pentaerythritols, sulphonic acid derivative, epoxy derivative, chlorinated paraffin, polymeric ester, glycerol triacetate, and glycerol diacetate, or a derivative or a mixture thereof_

12. The laminated tissue construct of claim 6, wherein said cultures mammalian cells form a tissue construct.

13. A biofilm for supporting support dependant cells comprising at least one milk protein selected in the group consisting of a globulin, a lactoglobulin or fragments thereof and a plasticizing quantity of at least one plasticizer.

14. The biofilm of claim 13, wherein said milk protein is a β-lactoglobulin.

15. The biofilm of claim 13, wherein said cells are selected from the group of fibroblasts, keratinocytes, muscular cells, skin cells, epithelial cells, mesenchymal cells, mesodermal cells, and adult stern cells.

16. The biofilm of claim 13, wherein said plasticizer is selected from the group consisting of glycerol, diethyleneglycol, polyethylen glycol, polybasic acid, phosphoric acid, fatty acid or substitute thereof, palmitate, stearate, oleate, ricinoleate, laurate, sorbitan monolaurate, sorbitan monooleate, poly(oxyethylene)-(20) sorbitan monolauratc, butyl acetyl ricinoleate, glycerol tricaprolate, glycerol tributyrate, triethylene glucol caprylate, polyglycol, polyoxyalkylene, pentaerythritols, sulphonic acid derivative, epoxy derivative, chlorinated paraffin, polymeric ester, glycerol triacetate, and glycerol diacetate, or a derivative or a mixture thereof.

## Patentansprüche

1. Verfahren zur IIerstellung eines laminierten Gewebekonstruktes, welches die folgenden Schritte aufweist:
a) In-Verbindung-Biringen eines wässrigen Kulturmediums, das Trägerabhängige Säugetierzellen enthält, mit einem Biofilm, der zumindest ein Milchprotein aufweist, das ausgewählt ist aus der Gruppe bestehend aus einem Molkenprotein, einem Glohulin, einem Lactoglobulin oder Fragmenten daraus, und mit einer weichmachenden Menge an zumindest einem Weichmacher, und
b) Kultivieren der Zellen auf dem Biofilm unter Bedingungen, die die Bildung einer Zellschicht ermöglichen, die an den Biofilm adhäriert, zur Bildung des laminierten Gewebekonstrukts.

2. Verfahren nach Anspruch 1, bei welchem der Biofilm zwischen 1 bis 80 % Milchprotein und zwischen 1 bis 50 % Weichmacher aufweist.

3. Verfahren nach Anspruch 1, bei welchem das Milchprotein ein β-Lactoglobulin ist.

4. Verfahren nach Anspruch 1, bei welchem die Zellen ausgewählt sind aus der Gruppe von Fibroblasten, Keratinocyten, Muskelzellen, Hautzellen, Epithelzellen, mesenchymale Zellen, mesodermale Zellen und adulte Stammzellen.

5. Verfahren nach Anspruch 1, bei welchem der Weichmacher ausgewählt ist aus der Gruppe bestehend Glycerol, Diethylenglycol, Polyethylenglycol, mehrprotonische Säure, Phosphorsäure, eine Fettsäure oder einem Ersatzstoff davon, Palmitat, Stearat, Oleat, Ricinoleat, Laurat, Sorbitan-Monolaurat, Sorbitan-Monooleat, Poly(oxyethylen)-(20)-Sorbitan-Monolaurat, Butylacetylricinoleat, Glyceroltricaprolat, Glyceroltributyrat, Triethylenglucolcaprylat, Polyglycol, Polyoxyalken, Pentaerythritole, Schwefelsäurederivat, Epoxyderivat, chloriertes Paraffin, Polymerester, Glyceroltriacetat und Glyceroldiacetat, oder ein Derivat oder Mischungen davon.

6. Laminiertes Gewebekonstrukt mit zumindest einer Schicht eines Biofilms, der zumindest ein Milchprotein aufweist, das ausgewählt ist aus der Gruppe bestehend aus einem Molkenprotein, einem Glubolin, einem Lactoglobulin oder Fragmenten davon, und einer weichmachenden Menge an zumindest einem Weichmacher, und zumindest eine Schicht an kultivierten Säugetierzellen, die an zumindest eine Seite des Biofilms adhärieren.

7. Laminiertes Gewebekonstrukt nach Anspruch 6, bei welchem die kultivierten Säugetierzellen eine subkonfluente Zellschicht oder eine konfluente Zellschicht bilden.

8. Laminiertes Gewebekonstrukt nach Anspruch 6, bei welchem der Biofilm zwischen 1 bis 80 % Milchprotein und zwischen 1 bis 50 % Weichmacher aufweist.

9. Laminiertes Gewebekonstrukt nach Anspruch 6, bei welchem das Milchprotein ein β-Lactoglobulin ist.

10. Laminiertes Gewebekonstrukt nach Anspruch 6, bei welchem die Zellen ausgewählt sind aus der Gruppe von Fibroblasten, Keratinocyten, Muskelzellen, Hautzellen, Epithelzellen, mesenchymale Zellen, mesodermale Zellen und adulte Stammzellen.

11. Laminiertes Gewebekonstrukt nach Anspruch 6, bei welchem der Weichmacher ausgewählt ist aus der Gruppe bestehend aus Glycerol, Diethylenglycol, Polyethylenglycol, mehrprotonische Säure, Phosphorsäure, einer Fettsäure oder einem Ersatzstoff davon, Palmitat, Stearat, Oleat, Ricinoleat, Laurat, Sorbitan-Monolaurat, Sorbitan-Monooleat, Poly(oxyethylen)-(20)-Sorbitan-Monolaurat, Butylacetylricinoleat, Glyceroltricaprolat, Glyceroltributyrat, Triethylenglucolcaprylat, Polyglycol, Polyoxyalken, Pentaerythritole, Schwefelsäurederivat, Epoxyderivat, chloriertes Paraffin, Polymerester, Glyceroltriacetat und Glyceroldiacetat, oder ein Derivat oder Mischungen davon.

12. Laminiertes Gewebekonstrukt nach Anspruch 6, bei welchem die kultivierten Säugetierzellen ein Gewebekonstrukt bilden.

13. Biofilm zur Unterstützung von Träger-abhängigen Zellen mit zumindest einem Milchprotein, das ausgewählt ist aus der Gruppe bestehend aus einem Globulin, einem Lactoglobulin oder Fragmenten davon, und einer weichmachenden Menge an zumindest einem Weichmacher.

14. Biofilm nach Anspruch 13, bei welchem das Milchprotein ein β-Lactoglobulin ist.

15. Biofilm nach Anspruch 13, bei welchem die Zellen ausgewählt sind aus der Gruppe von Fibroblasten, Keratinocyten, Muskelzellen, Hautzellen, Epithelzellen, mesenchymale Zellen, mesodermale Zellen und adulte Stammzellen.

16. Biofilm nach Anspruch 13, bei welchem der Weichmacher ausgewählt ist aus der Gruppe bestehend aus Glycerol, Diethylenglycol, Polyethylenglycol, mehrprotonische Säure, Phosphorsäure, einer Fettsäure oder einem Ersatzstoff davon, Palmitat, Stearat, Oleat, Ricinüleat, Laurat, Sorbitan-Monolaurat, Sorbitan-Monooleat, Poly(oxyethylen)-(20)-Sorbitan-Monolauzat, Butylacetylricinoleat, Clyceroltricaprolat, Glyceroltributyrat, Triethylenglucolcaprylat, Polyglycol, Polyoxyalken, Pentacrythritole, Schwefelsäurederivat, Epoxyderivat, chloriertes Paraffin, Polymerester, Glyceroltziacetat und Glyceroldiacetat, oder ein Derivat oder Mischungen davon.

## Revendications

1. Procédé de production d'une construction en tissu stratifiée, comprenant les étapes consistant à :
a) mettre en contact un milieu de culture aqueux contenant des cellules de mammifère dépendantes d'un support avec un film biologique comprenant au moins une protéine du lait choisie dans le groupe consistant en une protéine lactosérique, une globuline, une lactoglobuline ou des fragments de celles-ci et une quantité de plastification d'au moins un plastifiant, et
b) mettre en culture lesdites cellules sur ledit film biologique dans une condition permettant la formation d'une couche de cellules mise à adhérer audit film biologique pour former la construction en tissu stratifiée.

2. Procédé selon la revendication 1, dans lequel ledit film biologique comprend entre 1 et 80 % de protéine du lait et entre 1 et 50 % de plastifiant.

3. Procédé selon la revendication 1, dans lequel ladite protéine du lait est une β-lactoglobuline.

4. Procédé selon la revendication 1, dans lequel lesdites cellules sont choisies dans le groupe des fibroblastes, des kératinocytes, des cellules musculaires, des cellules de la peau, des cellules épithéliales, des cellules mésenchymateuses, des cellules mésodermiques et des cellules souches adultes.

5. Procédé selon la revendication 1, dans lequel ledit plastifiant est choisi dans le groupe consistant en le glycérol, le diéthylène glycol, le poly(éthylène glycol), l'acide polybasique, l'acide phosphorique, un acide gras ou un substitut de celui-ci, un palmitate, un stéarate, un oléate, un ricinoléate, un laurate, le monolaurate de sorbitan, le monooléate de sorbitan, le poly(monolaurate d'oxyéthylène-(20) sorbitan), le ricinoléate de butyl acétyle, le tricaprolate de glycérol, le tributyrate de glycérol, le caprylate de triéthylène glycol, le poly(glycol), un poly(oxyalkyléne), les pentaérythritols, un dérivé d'acide sulfonique, un dérivé d'époxy, la paraffine chlorée, un ester polymère, le le triacétate de glycérol, et le diacétate de glycérol ou un dérivé ou un mélange de ceux-ci.

6. Construction en tissu stratifiée comprenant au moins une couche de film biologique comprenant au moins une protéine du lait choisie dans le groupe consistant en une protéine lactosérique, une globuline, une lactoglobuline ou des fragments de celles-ci et une quantité de plastification d'au moins un plastifiant, et au moins une couche de cellules de mammifère de culture mises à adhérer sur au moins un côté audit film biologique.

7. Construction en tissu stratifiée selon la revendication 6, dans laquelle lesdites cellules de mammifère de culture forment une couche de cellules sous-confluente ou une couche de cellules confluente.

8. Construction en tissu stratifiée selon la revendication 6, dans laquelle ledit film biologique comprend entre 1 et 80 % de protéine du lait et entre 1 et 50 % de plastifiant.

9. Construction en tissu stratifiée selon la revendication 6, dans laquelle ladite protéine du lait est une β-lactoglobuline.

10. Construction en tissu stratifiée selon la revendication 6, dans laquelle lesdites cellules sont choisies dans le groupe des fibroblastes, des kératinocytes, des cellules musculaires, des cellules de la peau, des cellules épithéliales, des cellules mésenchymateuses, des cellules mésodermiques et des cellules souches adultes.

11. Construction en tissu stratifiée selon la revendication 6, dans laquelle ledit plastifiant est choisi dans le groupe consistant en le glycérol, le diéthylène glycol, le poly(éthylène glycol), un acide polybasique, l'acide phosphorique, un acide gras ou un substitut de celui-ci, un palmitate, un stéarate, un oléate, un ricinoléate, un laurate, le monolaurate de sorbitan, le monooléate de sorbitan, le poly(monolaurate d' oxyéthylène-(20) sorbitan), le ricinoléate butyl acétyle, le tricaprolate de glycérol, le tributyrate de glycérol, le caprylate de triéthylène glycol, un poly(glycol), un poly(oxyalkylène), les pentaérythritols, un dérivé d'acide sulfonique, un dérivé d'époxy, la paraffine chlorée, un ester polymère, le triacétate de glycérol et le diacétate de glycérol, ou un dérivé ou un mélange de ceux-ci.

12. Construction en tissu stratifiée selon la revendication 6, dans laquelle lesdites cellules de mammifère de culture forment une construction en tissu.

13. Film biologique pour supporter des cellules dépendant d'un support comprenant au moins une protéine du lait choisie dans le groupe consistant en une globuline, une lactoglobuline ou des fragments de celles-ci et une quantité de plastification d'au moins un plastifiant.

14. Film biologique selon la revendication 13, dans lequel ladite protéine du lait est une β-lactoglobuline.

15. Film biologique selon la revendication 13, dans lequel lesdites cellules sont choisies dans le groupe des fibroblastes, des kératinocytes, des cellules musculaires, des cellules de la peau, des cellules épithéliales, des cellules mésenchymateuses, des cellules mésodermiques et des cellules souches adultes.

16. Film biologique selon la revendication 13, dans lequel ledit plastifiant est choisi dans le groupe consistant en le glycérol, le diéthylène glycol, le poly(éthylène glycol), un acide polybasique, l'acide phosphorique, un acide gras ou un substitut de celui-ci, un palmitate, un stéarate, un oléate, un ricinoléate, un laurate, le monolaurate de sorbitan, le monooléate de sorbitan, le poly(monolaurate d'oxyéthylène-(20) sorbitan), le ricinoléate de butyl acétyle, le tricaprolate de glycérol, le tributyrate de glycérol, le caprylate de triéthylène glycol, un poly(glycol), un poly(oxyalkylène), les pentaérythritols, un dérivé d'acide sulfonique, un dérivé d'époxy, la paraffine chlorée, un ester polymère, le triacétate de glycérol et le diacétate de glycérol, ou un dérivé ou un mélange de ceux-ci.
